# EUROPÄISCHE PATENTSCHRIFT

(11) **EP 0 641 547 B1**
(45) Veröffentlichungstag und Bekanntmachung des Hinweises auf die Patenterteilung: **19.05.1999**
(21) Anmeldenummer: 94111015.7
(22) Anmeldetag: 15.07.1994
(51) Int. Cl.: A61B 17/56, A61F 2/44

(54) **Implantationssystem zur Wirbelkörperverblockung**
Implantation system for vertebral fusion
Système d'implantation pour la fusion vertébrale

(30) Priorität: 26.08.1993 DE 4328690
(43) Veröffentlichungstag der Anmeldung: 08.03.1995
(73) Patentinhaber: MAN Ceramics GmbH, 94453 Deggendorf (DE)
(72) Erfinder: Bertagnoli,Rudolf Dr.med., D-37073 Göttingen (DE)
(74) Vertreter: Viering, Jentschura & Partner

(56) Entgegenhaltungen:
- EP-A- 0 307 241
- WO-A-90/00037
- DE-A- 3 505 567
- US-A- 3 486 505
- US-A- 5 015 247

## Beschreibung

Die Erfindung bezieht sich auf ein Implantationssystem mit einem Implantat zur Wirbelkörperverblockung und mit einem langgestreckten Führungsinstrument.

Aus DE 35 05 567 ist ein System dieser Art bekannt, das aus einem Implantat und einem Instrument zum Einführen des Implantats besteht. Das Implantat besteht aus einem vollen Zylinder mit einem Außengewinde, das mittels Spannbacken eines Implantierinstruments an einem Ende fest einspannbar ist. Die in dem Instrument axial verschiebbaren Spannbacken besitzen an der Innenseite scharfkantige Leisten, die sich in das Implantat eindrücken lassen, um das Implantat gegen Drehen zu sichern. Mit dem bekannten Instrument wird das Implantat in einen vorbereiteten Hohlraum zwischen zwei benachbarte Wirbelkörper eingeschraubt, was eine komplizierte Operation erfordert.

Aus der EP 179 695 ist ein aus einer einfachen Scheibe ausgebildetes Implantat gezeigt, das zwar einfacher implantiert werden kann, das aber zur Lagesicherung Laschen aufweist, über die das Implantat am Wirbelknochen angeschraubt wird. Der Schraubvorgang erfordert jedoch eine längere Handhabung.

Der Erfindung liegt die Aufgabe zugrunde, ein Implantationssystem zur Wirbelverblockung zu entwickeln, das einen raschen Implantationsprozeß und eine sichere Wirbelverblockung ermöglicht.

Die Aufgabe wird erfindungsgemäß durch die Merkmale des Anspruchs 1 gelöst

Das Implantat, das außer den anatomisch und mechanisch bedingten Eigenschaften Führungsmittel enthält, ermöglicht z. B. in Kooperation mit dem entsprechenden Führungswerkzeug eine rasche und exakte Plazierung des Implantats. Mit einem langgestreckten Führungswerkzeug kann bei geringer Querschnittsfläche das Werkzeug durch das Gewebe bis zur Implantationsstelle eingeführt werden. Das bedeutet, daß es mit dem erfindungsgemäßen, führbaren Implantat außerdem möglich sein wird, eine Implantation ohne große Schnittwunden am Patienten durchzuführen. Das gibt die Möglichkeit, ambulante Operationen für Wirbelverblockungen zu unternehmen.

Ein weiterer Vorteil des erfindungsgemäßen Implantationssystems liegt darin, daß Probeimplantate gleicher Konfiguration aus Metall eingesetzt werden können, um beispielsweise mit Diagnosegeräten intraoperativ die Position zu kontrollieren, wenn das endgültige Implantat z. B. aus Kunststoff besteht und keine Röntgenkontrastmittel aufweist.

Das Implantat ist ein einstückiges, volles oder zum Teil hohles Baute, das im Prinzip jede anatomisch zweckmäßige Formgebung haben kann. Die Formgebung richtet sich nach der Anatomie, nach der Zweckmäßigkeit eines Einführungsinstruments und/oder nach Fertigungsgesichtspunkten des Implantats. Das Implantat besteht vorzugsweise aus faserverstärktem Material, insbesondere kohlenstoffaserverstärktem Kunststoff.

Im allgemeinen wird das Implantat die Wirbelkörper auf Dauer zusammenfügen. Aber auch zur temporären Stützfunktion geeignete oder resorbierbare Implantate lassen sich gemäß der Erfindung ausbilden.

Das Implantat kann gemäß einer fertigungstechnisch einfachen Ausführung ein voller oder hohler Quader oder Zylinder sein, der in Längsrichtung mindestens eine Führungsnut und/oder Führungsfeder aufweist.

Aus der US 4,834,757 sind quaderförmige, hohle oder volle Wirbelverblockungen bekannt, die aber nur Löcher zur Aufnahme eines Instruments aufweisen, mit dem das Implantat ohne Führung eingesetzt wird, wobei Verkantungen nicht auszuschließen sind. Aus der US 3,486,505 ist ein Implantationssystem mit einem Implantat zur Wirbelkörperverblockung und mit einem langgestreckten Führungsinstrument bekannt, das die Form einer Pinzette mit seitlichen Laschen hat, bei dem das Implantat per Hand seitlich eingesetzt und in Längsrichtung zwischen die Laschen geschoben werden muß, bevor es mit einem axialen Dorn in den Hohlraum der Wirbelkörper eingedrückt wird.

Insbesondere für Halswirbel eignen sich auch bogen- oder U-förmige Implantate, deren freie Schenkel bzw. Seitenteile die Führungsmittel aufweisen. Ein derartiges Implantat kann liegend oder hochkant, d. h. mit jedem Schenkel oder Seitenteil, einen Wirbelkörper kontaktierend, eingesetzt werden. Gemäß einer weiteren Ausgestaltung der Erfindung sind die U-förmigen Implantate elastisch ausgebildet, so daß sie zur Selbstfixierung mit Vorspannung eingesetzt werden.

Zur Fixierung und Förderung des Anwachsens sind die Implantate an den mit den Wirbelkörpern zusammenwirkenden Flächen strukturiert, aufgerauht und/oder beschichtet und/oder mit Öffnungen versehen, durch die Knochenmaterial in Kontakt mit den angrenzenden Wirbelkörpern kommen kann.

Das erfindungsgemäße System erleichtert und beschleunigt einen Implantationsprozeß, wobei es bei Anwendung des langgestreckten Führungsinstruments auch eine geringe Länge des Hautschnitts ermöglicht, indem das langgestreckte Führungsinstrument mit relativ geringem Querschnitt gleichzeitig zur Verdrängung von Muskelgewebe dienen bzw. ausgebildet werden kann, so daß sämtliche Operationsschritte innerhalb des langgestreckten Instruments erfolgen.

Beim Operationsvorgang wird das Führungsinstrument durch einen relativ geringen Schnitt bis zum Anschlag an die zu behandelnden Wirbelkörper oder dazwischen geführt. Dieses Führungsinstrument dient als Zugang, Halterung und Führung für die weiteren Implantationsvorgänge. Mit passenden Bearbeitungswerkzeugen wird der Hohlraum für das oder die Implantate vorbereitet und anschließend, ohne das Führungsinstrument abzusetzen, das Implantat paßgenau in den vorbereiteten Hohlraum eingeführt. Danach und gegebenenfalls nach Einbringung von Knochenmaterial und eines Knochendeckels zum Verschluß wird das Führungsinstrument wieder entfernt und die Wunde verschlossen.

Gemäß einer anderen Ausgestaltung ist das Implantat mit Öffnungen und/oder Stegen ausgerüstet, die eine Verankerung des Implantats mit in den Knochen einschlagbaren Krampen ermöglicht. Das Implantat kann dabei nach anatomischen Gesichtspunkten und rascher Handhabung optimiert werden, ohne dabei die Fixierungsmöglichkeiten berücksichtigen zu müssen. Die Krampen bieten nämlich eine freie Wahl der Anordnung der Fixierungsmittel.

Ein vorstehend beschriebenes quaderförmiges Implantat erhält zu diesem Zweck beispielsweise, ausgehend von einer Stirnseite, Durchbrüche, durch die ein Schenkel einer oder mehrerer Krampen durchgreift. Ein U-förmiges Implantat kann mit einem die freien Enden der beiden Implantatschenkel verbindenden Steg ausgestattet werden, der von den Krampen umklammert werden kann.

Die Fixierung mittels Krampen ist für alle Arten von konventionellen als auch neuartigen Implantaten für die Wirbelsäule möglich. Die Fixierung mittels Krampen hat gegenüber den Laschen gemäß EP 179 695 den Vorteil, daß keine Verschraubungen notwendig sind und daß sie viel mehr Variationsmöglichkeiten der Position der Befestigung bietet.

Die Krampen können vorteilhafterweise mit einer Schnappwirkung ausgebildet sein, deren Schenkel beim Darüberschieben um den Steg auseinandergedrückt werden und wieder zusammengehen, sobald die Engstelle den Steg überwunden hat. Auf diese Weise wirkt eine Spannung zwischen Krampenschenkel und Knochen zur besseren Verankerung der Krampen im Knochen. Außerdem wird eine sichere Fixierung der Krampen am Steg des Implantats gewährleistet.

Für die Verankerung können bekannte zwei- oder mehrschenklige, drahtförmige oder breitbandige Krampen (DE 33 10 835, GB 2,017,502) verwendet werden. Insbesonders eignet sich eine dreischenklige Krampe in Verbindung mit einer zweischenkligen Krampe, wobei die zwischenliegende Krampe durch eine mittige Aussparung am gebogenen Bereich der dreischenkligen Krampe durchgeführt wird. Die beiden Krampen werden in je einen der angrenzenden Wirbel eingeschlagen. Die Länge der Krampenschenkel ist bei schräger Einführung ungleich, weil sie bis zum Erreichen des Knochens unterschiedliche Strecken überwinden müssen.

Für die Krampen können Vorbereitungsdorne sowie ein Krampenhalter verwendet werden. Beide Werkzeuge sind S-förmig und an einem freien Ende gabelartig ausgebildet. Das zweite Ende ist zur Aufnahme eines Stößels ausgestaltet.

Die Erfindung wird anhand von in der Zeichnung schematisch dargestellten Ausführungsbeispielen näher erläutert. Es zeigen:
Fig.1 bis 5 je ein Ausführungsbeispiel eines Implantats,
Fig.6 ein Führungsinstrument,
Fig.7 den Querschnitt eines weiteren Führungsinstruments,
Fig.8 ein Bearbeitungswerkzeug,
Fig.9 und 10 je ein Ausführungsbeispiel für Implantatsicherung mittels Krampen und
Fig.11 und 12 je ein Werkzeug für Krampen.

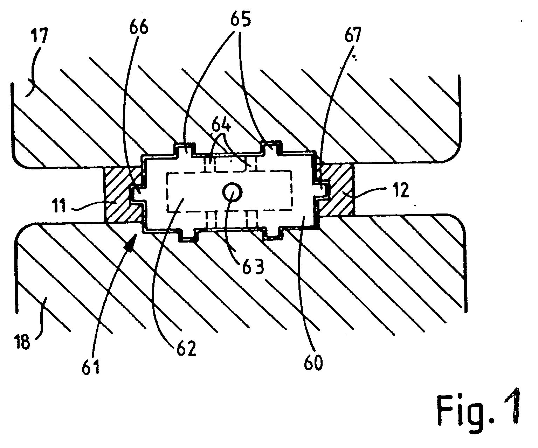

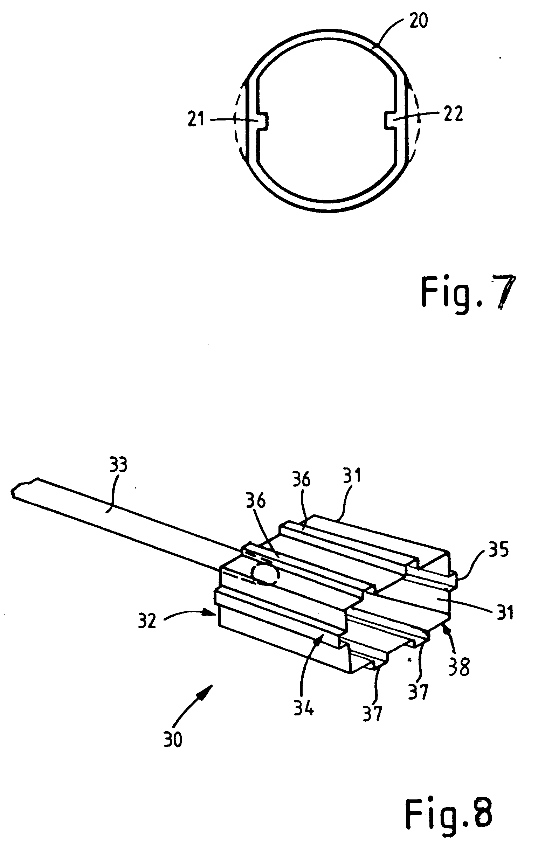

Fig. 1 zeigt ein erstes Implantats 60, das in der Vollendung einer Verblockung von zwei Wirbelkörpern 17, 18 im Längsschnitt der Wirbelsäule dargestellt ist. Zwischen den Wirbelkörpern 17, 18 befindet sich das Implantat 60, das in einen Hohlraum 61 eingebracht wurde, der zuvor unter Verwendung eines Stanzwerkzeugs 30 (Fig. 8) ausgearbeitet wurde. Das Implantat 60 ist ein quaderförmiges Bauteil mit einem hohlen Innenraum 62, in den über eine Öffnung 63 Knochenmaterial eingeführt werden kann. Das Knochenmaterial kommt über Bohrungen 64, die die obere und untere Wandung des Implantats 60 durchqueren, mit den Wirbelkörpern 17, 18 in Kontakt, so daß eine Verwachsung von einem Wirbelkörper 17 zum anderen Wirbelkörper 18 möglich ist. Als Federn ausgebildete Führungsmittel 65 an den Kontaktflächen zwischen dem Implantat 60 und den Wirbelkörpern 17 und 18 dienen zur sicheren Verankerung des Implantats 60 zwischen den benachbarten Wirbelkörpern 17,18. Die Formgebung der Führungsmittel 65 werden bei der Herstellung des Hohlraumes 61 unter Verwendung des zugehörigen Werkzeugs, in diesem Fall des Stanzwerkzeugs 30, gleich berücksichtigt. Das Implantat kann auch aus resorbierbarem Material bestehen, so daß dessen anfängliche Stützwirkung später vom Knochenmaterial übernommen wird.

Die Führungsmittel 65 und/oder seitliche, ebenfalls als Federn 66,67 ausgebildete Führungsmittel werden zur geführten Einsetzung des Implantats 60 in den vorbereiteten Hohlraum 61 der Wirbelkörper 17,18 verwendet.

Mit Hilfe der Führungsfedern 66 und 67 und von Führungsschienen 11 und 12, die weiter unten beschrieben werden, wird das Implantat nämlich sehr leicht und paßgenau in den vorbereiteten Hohlraum 61 eingeführt.

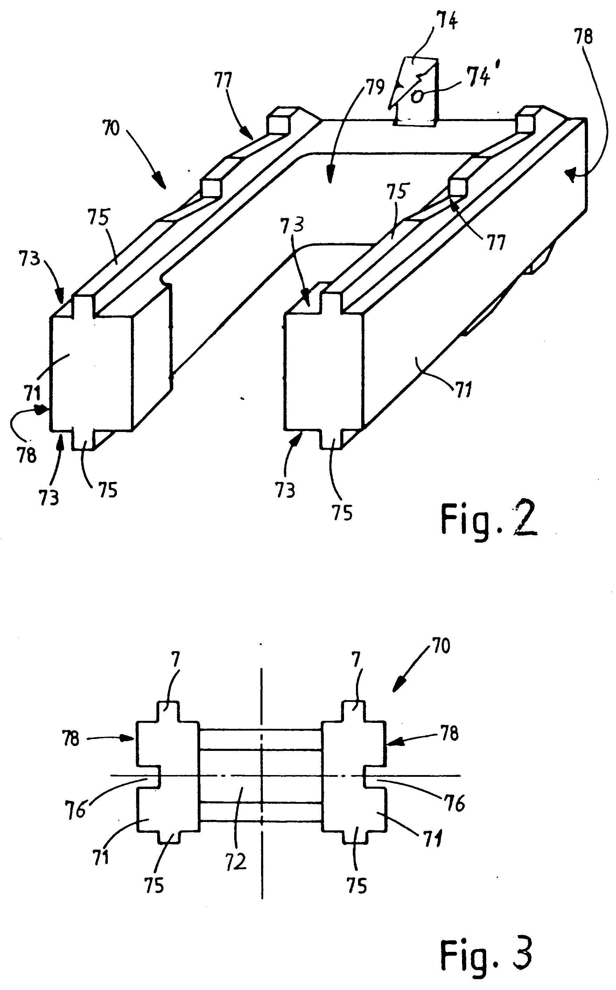

In Fig. 2 ist ein anderes Beispiel eines Implantats 70 gezeigt, das insbesondere für die Verblockung von Halswirbelkörpern geeignet ist. Das Implantat 70 besteht aus einem U-Bogen, dessen beiden Schenkel 71 an jeder mit einem Wirbel zusammenwirkenden Fläche 73 eine Unebenheit in Form von einer längsgerichteten Feder 75 hat, die gleichzeitig als Führungsfedern dienen können. Die Führungsfedern 75 erlauben eine sichere laterale Verankerung des Implantats 70. Um auch eine sichere Verankerung in sagittaler Ebene zu gewährleisten, können die Federn 75 Einschnitte 77 aufweisen, in die Knochenmaterial hineinwachsen kann. Diese Einschnitte können beispielsweise auch bei den Unebenheiten 65 des Implantats 60 vorgesehen werden.

Zur geführten Implantation des in Fig. 2 gezeigten Implantats 70 kann ein rohrförmiges oder ein aus Führungsschienen bestehendes Instrument verwendet werden, das vier Führungsnuten aufweist, die mit den Führungsfedern 75 des Implantats 70 zusammenwirken. Die Führung kann, wie in Fig. 3 gezeigt ist auch auf die Seiten 78 des Implantats 70 verlagert werden. Hier ist an den Außenseiten 78 der Schenkel 71 je eine Führungsnut 76 vorgesehen. Der Zwischenraum 79 zwischen den Schenkeln 71 des Implantats 70 kann mit Knochenmaterial gefüllt werden, das vor dem Einsetzen des Implantats 70 in den Zwischenraum 79 eingepreßt wird.

Die oben beschriebenen Implantate haben eine konstante Höhe. Die Höhe kann aber auch sich konisch vergrößernd ausgelegt sein, um mit dem Implantat einen Spreizvorgang durchzuführen, damit die physiologischen Segmentkrümmungen wieder hergestellt werden.

Dazu wird im Operationsgebiet der Wirbelkörper ein Hohlraum vorbereitet, dessen konstanter Querschnitt dem kleinsten bis mittleren Querschnitt des Implantats entspricht. Das Implantat wird dann mit Schlagstößen zwischen die Wirbelkörper eingeführt. Dieser Prozeß kann, innerhalb und mittels eines Führungsinstrumentes durchgeführt werden.

Eine laterale Spannung zwischen Implantat und Knochen läßt sich aus Fixierungsgründen ebenfalls realisieren. Das U-förmige Implantat kann beispielsweise durch Zusammendrücken der Schenkel 71 mit Vorspannung eingesetzt werden. Die dadurch erreichte hohe Reibung zwischen Implantat und Knochen unterstützt die sichere Verankerung des Implantats.

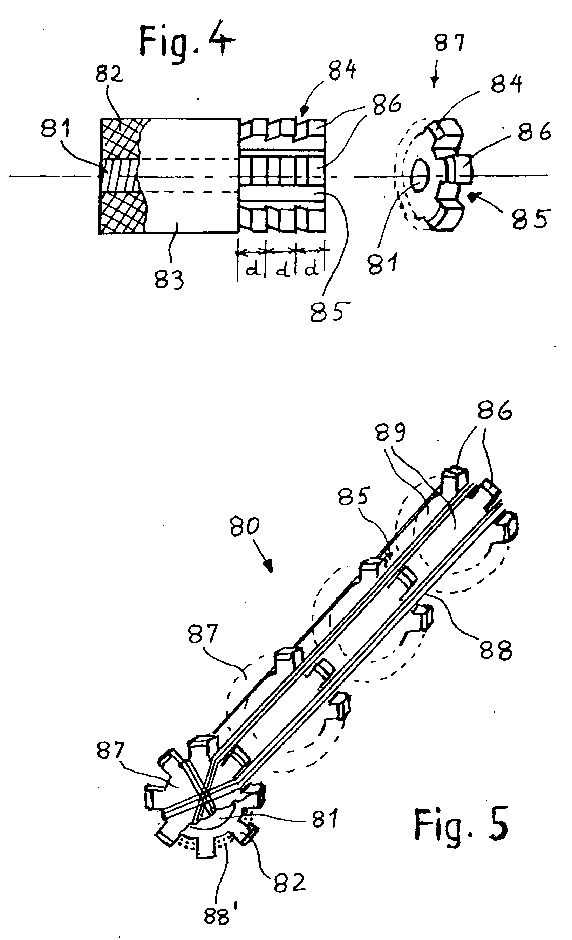

Die vorstehend beschriebenen Implantate haben im wesentlichen rechteckige Querschnitte. Es ist selbstverständlich auch möglich, Implantate zu verwenden, die mehr oder weniger runde Querschnitte mit entlang der Mantellinien verlaufenden Führungsmitteln aufweisen. Ein Ausführungsbeispiel hierzu ist in den Fig. 4 und 5 gezeigt.

In Fig. 4 und 5 sind verschiedene Herstellungsphasen eines zylindrischen Implantats 80 gezeigt, das aus Faserverbundwerkstoff besteht und käfigartig ausgebildet ist. Ein zylindrischer Kern 81 wird mit mehreren Flechtwerklagen 82 umgeben oder mit mehreren Kreuzlagen umwickelt. An dem so hergestellten Zylinder 83 werden in definierten Abständen d in Umfangsrichtung verlaufende Einkerbungen 84 und in Längsrichtung verlaufende Nuten 85 durch Schleifen ausgearbeitet, so daß der Außenumfang des Zylinders 83 aus zahlreichen Zähnen 86 besteht. Der Zylinder 83 wird anschließend in d-dicke Scheiben 87 aufgetrennt, die die Stützen für das käfigartige Implantat 80 bilden.

Wie in Fig. 5 näher gezeigt ist, werden die Stützscheiben 87 zum Umwickeln mit UD-Fasern 88 auf einem dünnen, nicht dargestellten Stahldorn in vorbestimmten Abständen positioniert. Die UD-Fasern 88 werden in die Nuten 85 zwischen den Zähnen 86 in Längsrichtung der von den Stützscheiben 87 gebildeten Achse über alle Stützscheiben gewickelt. Die UD-Faserlagen werden bis unterhalb der Zähne 86 gewickelt, so daß die Zähne 86 mit der Abschrägung 84 als Widerhaken für die Verankerung und gleichzeitig als Führung im Implantatinstrument dienen. Der Innenraum des Implantats wird mit Knochenmaterial gefüllt, so daß ein Durchwachsen von einem Wirbelkörper, durch die Zwischenräume 89 zwischen den UD-Faserbündeln 88', zum anderen benachbarten Wirbelkörper möglich ist.

Das Implantat 80 gemäß Fig. 4, 5 stellt eine Ausführung eines käfigartigen Implantats dar, das in einer fertigungstechnisch einfachen Art im Faserwickelverfahren herstellbar ist und das die Vorteile von unter Zugspannung und mit gewünschter Orientierung gelegten Fasern hat. Ein derartiges Implantat 80 eignet sich insbesondere für die Lendenwirbelverblockung.

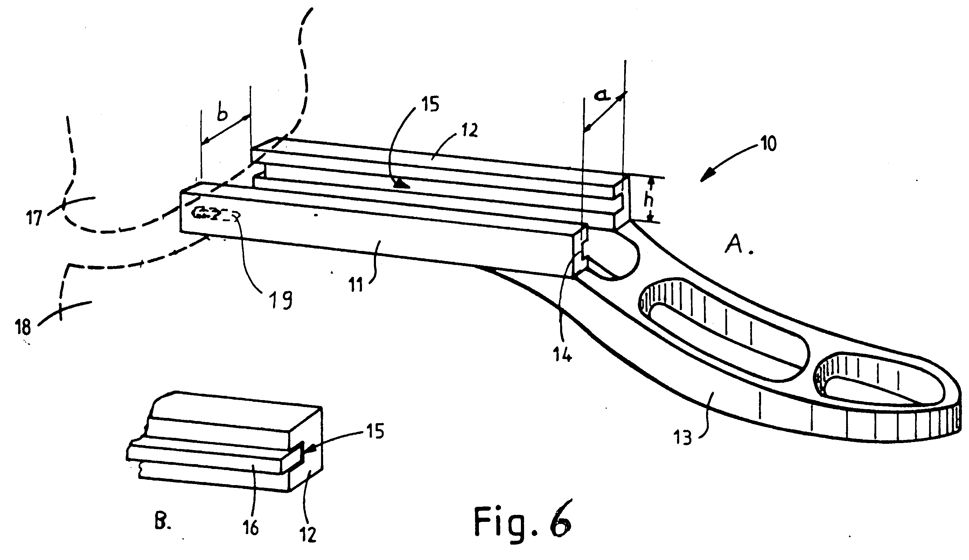

In Fig. 6A ist eine als Führungsinstrument 10 ausgebildete Hilfseinrichtung zur Durchführung von Wirbelkörperverblockungs-Implantationen gezeigt, die im wesentlichen aus zwei Führungsschienen 11 und 12, die parallel zueinander orientiert und starr mit einem Griff 13 verbunden sind, besteht. Die Führungsschienen 11 und 12 sind langgestreckte Quader mit jeweils einer Längsnut 14, 15 in den sich gegenüberliegenden Seiten der Führungsschienen 11, 12. Die Längsnuten 14 und 15 dienen zur Führung von Bearbeitungswerkzeugen und Implantaten, die jeweils die dazu passenden Führungsfedern aufweisen.

Das aus zwei nebeneinander gelagerten Führungsschienen 11 und 12 ausgebildete Instrument 10 gemäß Fig. 6 kann in der Höhe h so ausgebildet werden, daß es zwischen zwei benachbarte Wirbelkörper 17 und 18 einführbar ist. Der nach oben oder unten verbleibende Freiraum zwischen den Führungsschienen 11 und 12 ermöglicht die Einführung von Bearbeitungswerkzeugen oder Implantaten unterschiedlicher Höhen, d. h., die Werkzeuge und Implantate sind lediglich in ihrer mit den Führungsfedern definierten Breitenabmessung a vorbestimmt und in der übrigen Breitenabmessung b begrenzt. Aber auch hier können Maßunterschiede berücksichtigt werden, wenn es zweckmäßig oder wünschenswert ist, was durch in die Längsnuten 14, 15 einschiebbare Einsätze leicht erfüllt werden kann. Fig. 6B zeigt beispielsweise einen als langgestreckten Quader ausgebildeten Einsatz 16, der die Führungsnut 15 in eine Führungsfeder 16 umwandelt, womit gleichzeitig eine Breitenänderung verbunden werden kann. Nimmt ein Werkzeug, z. B. ein Bohrer (der einen Hohlraum geringerer Breite in den Wirbelkörpern herstellt), die volle Breite b des Führungsinstruments 10 ein, dann werden die Führungsnuten 14, 15 genutzt. Das in den Hohlraum einzusetzende Implantat dagegen wird die Breite b des Führungsinstruments 10 nicht ausfüllen. Um in solch einem Fall das Implantat nicht mit hohen Führungsfedern ausrüsten zu müssen, wird man entweder den Nutenabstand a mit einem entsprechenden Einsatz verringern oder, wie in Fig. 6 gezeigt, Federn 16 bilden, die mit entsprechenden Nuten am Implantat zusammenwirken können.

Auf diese Weise kann ein und dasselbe Führungsinstrument 10 mit Führungsnuten 14, 15 (Fig. 6A) oder mit Führungsfedern 16 (Fig. 6B) und für unterschiedliche Breiten a und b ausgestattet sein. Es ist selbstverständlich möglich, die Führungsschienen 11 und 12 einstückig mit Führungsfedern auszubilden.

Das in Fig. 6 gezeigte, aus Führungsschienen 11, 12 gebildete Instrument ist fertigungstechnisch sehr einfach herstellbar und benötigt einen sehr geringen Raum bei der Operation. Das Führungsinstrument 10 eignet sich für ambulante Implantationen, bei denen die gesamte Operation durch ein vorher eingeführtes, das Gewebe verdrängendes Rohr durchgeführt wird. Durch dieses Rohr können die Führungsschienen 11 und 12 bis zu den Wirbelkörpern 17, 18 gebracht werden.

Es ist auch denkbar, daß das eingeführte Rohr die Führungsfunktion übernimmt, d. h., daß das Führungsinstrument für die Implantation nicht, wie in Fig. 6, aus Schienen, sondern als ein nahezu geschlossenes Rohr 20 ausgebildet ist. Fig. 7 zeigt den Querschnitt eines rohrförmigen Führungsinstrumentes. Innerhalb des Führungsrohres 20 sind zwei Führungsfedern 21 und 22 vorgesehen, die mit entsprechenden Führungsnuten der Werkzeuge und Implantate zusammenwirken. Es sind selbstverständlich auch andere Querschnitte des Führungsinstruments möglich, soweit diese anatomisch und fertigungstechnisch sinnvoll sind. Der Außenumfang ist zylindrisch, gegebenenfalls mit Abflachungen, wie in Fig. 2 gezeigt ist.

In Fig. 8 ist ein als Meißel dienendes Stanzwerkzeug 30 gezeigt, das zur Aushebung des Hohlraumes 61 mit vieleckigem Querschnitt ausgebildet ist. Das Stanzwerkzeug 30 besteht aus einem hohlzylindrischen Meißel 31 mit rechteckiger Schneide 38 an einem Ende und mit geschlossenem zweiten Ende 32. Die geschlossene Stirnseite 32 trägt eine Schlagstange 33. An den Seitenflächen des Meißels 31 sind Führungsfedern 34 und 35 eingeformt, die in die Führungsnuten 14 bzw. 15 des Führungsinstruments 10 hineingreifen.

Bei dem in Fig. 8 dargestellten Stanzwerkzeug 30 sind auch in der oberen und unteren Seite des Meißels 31 Längsfedern 36 und 37 eingeformt. Mit diesen Federn 36 und 37 werden entsprechende Nuten in die Wirbelkörper 17 und 18 eingemeißelt, die zur sicheren Plazierung und Halterung der entsprechend ausgebildeten Implantats dienen. Die Konfiguration der oberen und unteren Fläche des Meißels 31 hat keinen Einfluß auf die Ausgestaltung des Führungsinstruments 10, zumal dessen Führungsschienen 11 und 12 lediglich mit den Seitenflächen des Meißels 31 in Verbindung kommen.

Bei einer Operation wird das Führungsinstrument mit der Stirnseite an die zu behandelnden Wirbelkörper zur Anlage gebracht und über in das Knochenmaterial eindringende Fixierstifte verankert. Anschließend wird ein Hohlraum für das Implantat vorbereitet. Dazu wird zunächst ein Implantat durch das Führungsinstrument zum Absaugen der Bandscheibe eingeführt. Je nach Formgebung des einzusetzenden Implantats wird als Knochenbearbeitungswerkzeug ein Bohrer, Meißel oder dergleichen verwendet und zur Räumung von Knochenmaterial vom Führungsinstrument geführt. Damit ergibt sich ein in seiner Formgebung gegenüber dem Führungsinstrument genau plazierter Hohlraum. Entstandene Knochenspäne werden abgesaugt. Anschließend wird - bei Bedarf - ein Probeimplantat aus Metall gleicher Formgebung wie der Hohlraum und das endgültige Implantat eingesetzt, um mittels eines Diagnosegerätes intraoperativ die Position zu kontrollieren.

Ein Probeimplantat ist in den Fällen erforderlich, in denen das endgültige Implantat aus Kunststoff besteht und keine Röntgenkontrastmarkierungen hat. Nach der Kontrolle und gegebenenfalls Nachbesserung der Hohlraumlage wird das eigentliche Implantat - ebenfalls über das Instrument geführt - in den formgenauen Hohlraum eingebracht.

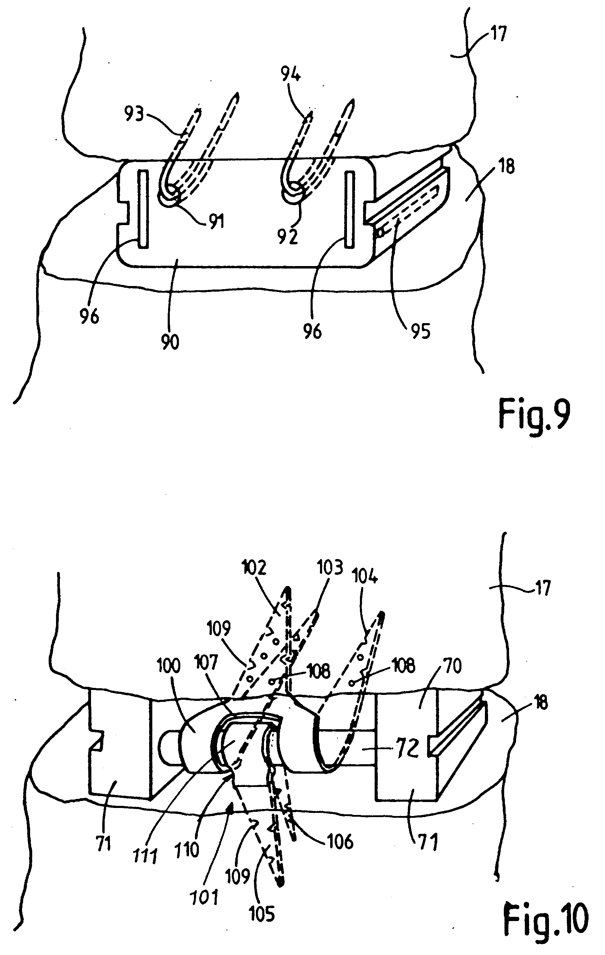

Zur Sicherung der Implantate im ersten Stadium mittels Krampen sind in den Fig. 9 und 10 zwei Beispiele gezeigt.

Fig. 9 zeigt zwei Wirbelkörper 17, 18, die mit einem quaderförmigen Implantat 90 verblockt sind. Durch zwei am oberen Ende des Implantats 90 befindliche Durchbrüche 91,92 ist jeweils ein Schenkel von jeweils einer haarnadelförmig gebogenen Drahtkrampe 93, 94 durchgeführt. Die Krampen 93, 94 sind in den angrenzenden oberen Wirbelkörper 17 schräg eingeschlagen und weisen unterschiedlich lange Schenkel auf, um eine gleiche Eindringtiefe in den Knochen zu haben. Der untere Schenke muß einen längeren Freiraum bis zum Wirbelknochen 17 überwinden, dieser Schenkel ist daher länger als der obere.

In Fig. 10 ist ein Beispiel mit zwei ineinandergreifenden Krampen 100 und 101 gezeigt, die aus gebogenen breiten Bändern hergestellt sind. Derartige Krampen 100, 101 eignen sich insbesondere für offene Implantate, die einen Steg aufweisen oder denen ein gesonderter Steg für die Krampen zugeordnet werden kann. Das U-förmige Implantat 70 gemäß Fig. 7a und b ist ein Beispiel hierfür. Für die Krampen 100, 101 ist ein gesonderter, die Implantatschenkel 71 verbindender Steg 72 vorgesehen, den die Krampen 100, 101 umgreifen. Die eine Krampe 100 ist dreischenkelig (102 bis 104) und mit einer zentralen Ausnehmung 107 im gebogenen Bereich ausgebildet derart, daß eine zweischenkelige zweite Krampe in dieser Ausnehmung 107 den Steg 72 umgreifen kann. Die Schenke 102 bis 107 laufen spitz zu und weisen Löcher 108 und seitliche Einkerbungen 109 als Knocheneinwachshilfen auf. Der obere einzelne Schenke 102 der dreischenkligen Krampe ist kürzer ausgebildet als die beiden unteren Schenkel 103, 104, die vom Steg 72 einen weiteren Weg zum Wirbelkörper 17 haben.

Die Krampe 101 hat zusätzlich eine Einschnappfunktion. Sie weist eine Einschnürung 110 auf, durch die die Schenkel 105 und 106 beim Einschlagen vom Steg 72 aufgespreizt werden und im aufgespreizten Zustand in den Knochen 18 eindringen. Nach Überwinden der Einschnürung 110 schnappt diese teilweise zu, so daß der Steg 72 im Bogenteil 111 der Krampe 101 eingeschlossen bleibt. Die zurückfedernden Schenkel 105, 106 stehen dadurch mit dem Knochen 18 unter gegenseitigem Druck, was die Verankerung der Schenkel im Knochen unterstützt.

Beim Beispiel gemäß Fig. 10 ist die eine Krampe 100 im oberen Wirbelkörper 17 und die andere Krampe im unteren Wirbelkörper 18 verankert. Es sind Kombinationen von Krampen unterschiedlicher Gestaltungen und Anordnungen möglich. Das erfindungsgemäße Implantat läßt sich auch mit integrierten Krampen ausrüsten. In Fig. 7a ist eine integrierte Krampe 74 gezeigt, die entweder direkt eingeschlagen wird oder mittels Schrauben befestigbar ist. Die Schraube wird durch die in der integrierten Krampe 74 vorgesehenen Bohrung 74' geführt.

Die Implantate 60, 70, die Krampen 90, 95 und Führungsinstrumente bestehen vorzugsweise aus faserverstärktem Kunststoff. Zum Lokalisieren der eingesetzten Implantate sind diese mit Kontrastmitteln ausgestattet. Dazu dienen Stäbe 95, 96 aus röntgenundurchlässigem Material, z.B. Metall oder Bariumsulfat, die, wie in Fig. 9 angedeutet ist, sich im Implantat (95) oder an der Implantatoberfläche (96) in definierter Orientierung befinden. Allerdings sind bei Kunststoff-Implantaten gemäß der Erfindung Röntgenkontrastmarkierungen nicht zwingend notwendig. Das erfindungsgemäße System erlaubt eine bequeme Anwendung von Probeimplantaten aus Metall, die nur zum Zwecke der Lagekontrolle in den vorbereiteten Knochenhohlraum eingesetzt und wieder herausgenommen werden.

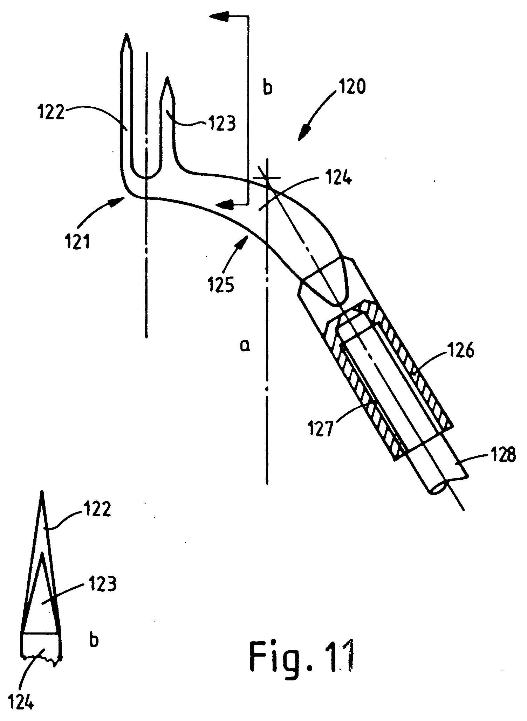

Um die Krampen, die insbesondere von der einfachen Drahtform (gemäß Fig. 9) abweichen, leichter einbringen zu können, kann ein Vorbereitungswerkzeug gemäß Fig. 11 vorgesehen werden. Aus anatomischen Gründen ist das Vorbereitungswerkzeug 120 S-förmig mit einem annähernd 120° gebogenen Ende 121. An diesem Ende sind Dorne122, 123 im rechten Winkel zum übrigen langgestreckten Werkzeugteil 124 vorgesehen, das den zweiten S-Bogen 125 bildend gekrümmt ist derart, daß das freie Ende 126 zur Einleitung von Einschlagstößen annähernd parallel zu den Dornen verläuft.

Die Dorne 122, 123 dienen zur Vorbereitung von Löchern in den Wirbelknochen, in die anschließend die Schenkel von Krampen eingeschlagen werden. Sie können die Form eines angespitzten Stiftes haben oder in ihrer Formgebung an die der einzuführenden Krampenschenkel angepaßt sein. Bei einer Anpassung an die Krampe 101 gemäß Fig. 10 würden die Dorne 122, 123 in der Seitenansicht b dreieckförmig sein, wie es in Fig. 11b gezeigt ist.

Das freie Ende des langgestreckten Werkzeugteils 124 weist eine Schraubbohrung 127 zur Aufnahme eines Stößels 128 auf.

Nach dem Einsetzen des Implantats (z. B. gemäß Fig. 10) wird das Führungsinstrument 10 entfernt und das Vorbereitungswerkzeug 120 mit den Dornen 122, 123 nach unten zeigend und den Steg 72 umgreifend angesetzt. Durch Schläge auf den Stößel 128 werden die Dorne 122, 123 in den unteren Wirbelkörper 18 eingeschlagen und wieder herausgezogen. Mit einem entsprechenden, drei Dorne aufweisenden Vorbereitungswerkzeug werden die Löcher im oberen Wirbelkörper 17 für die zweite Krampe 100 hergestellt. Dieser Vorgang läßt sich durch den formgebundenen leichten Zugang zur Operationsstelle leicht und rasch durchführen.

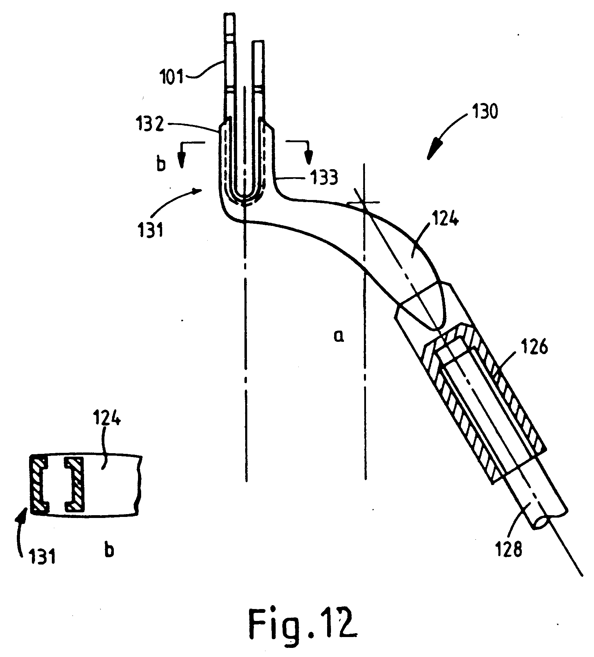

Anschließend lassen sich die Krampen 100, 101 ebenso leicht mit Hilfe einer Halterung nach Fig. 12 einführen. Die Krampenhalterung 130 hat eine ähnliche S-Formgebung wie das Vorbereitungswerkzeug 120. Es ist lediglich anstelle der Dorne 122, 123 eine U-förmige Halterung 131 vorgesehen, deren Querschnitt beispielsweise für die Krampe 101 nach Fig. 10 in Fig. 12b gezeigt ist. Die Krampe 101 wird in die U-förmige Halterung 131 eingelegt, worin sie durch Reibschluß hängen bleibt. Die Schenkel 132, 133 der U-förmigen Halterung 131 sind so kurz, daß sie beim Einschlagen der Krampen 101 gerade nicht in den Wirbelkörper 18 eindringen.

Die Werkzeuge 120, 130 für die Krampen bestehen vorzugsweise aus einem langgestreckten Werkzeugteil 124 mit zugehörigem Stößel 128 und einem Satz von Einsteckteilen mit unterschiedlichen Dornen bzw. Halterungen für verschiedenartige Krampen.

## Patentansprüche

1. Implantationssystem mit einem Implantat (60, 70, 80) zur Wirbelkörperverblockung, das an seiner Außenseite mit Federn (66, 67, 75) und/oder Nuten (76, 85) versehen ist, die in Längsrichtung des Implantats (60, 70, 80) verlaufen, und einem langgestreckten Führungsinstrument (10, 20), das an die Federn (66, 67, 75) und/oder die Nuten (76, 85) des Implantats (60, 70, 80) angepaßte Längsnuten (15) bzw. Längsfedern (16, 21, 22) aufweist, wobei das Implantat (60, 70, 80) mit seinen Federn (66, 67, 75) und/oder Nuten (76, 85) von den zugeordneten Längsnuten (15) bzw. Längsfedern (16, 21, 22) des Führungsinstruments (10, 20) geführt in einen vorbereiteten Hohlraum (61) zwischen benachbarten Wirbelkörpern (17, 18) hineinschiebbar ist.

2. Implantationssystem nach Anspruch 1, wobei das Implantat (60, 80) quaderförmig oder zylinderförmig ausgebildet ist.

3. Implantationssystem nach Anspruch 1 oder 2, wobei das Implantat (70) U-förmig ausgebildet ist und die Nuten (76) und/oder die Federn (75) an seinen freien U-Schenkeln (71) ausgebildet sind.

4. Implantationssystem nach einem der Ansprüche 1 bis 3, wobei das Führungsinstrument (10, 20) in Form zweier parallel zueinander verlaufender und zu einem Bauteil zusammengefaßter Führungsschienen (11, 12) oder in Form eines zylindrischen Rohrs (20) ausgebildet ist.

5. Implantationssystem nach einem der Ansprüche 1 bis 4, wobei die Längsfedern (16) an dem Führungsinstrument (10) auswechselbar angeordnet sind.

## Claims

1. Implantation system comprising an implant (60, 70, 80) for the immobilization of vertebrae, said implant being provided on its exterior with keys (66, 67, 75) and/or grooves (76, 85) which run in longitudinal direction of the implant (60, 70, 80), and an elongated guide instrument (10, 20) which comprises longitudinal grooves (15) or longitudinal keys (16, 21, 22), respectively, which are adapted to the keys (66, 67, 75) and/or the grooves (76, 85) of the implant (60, 70, 80), wherein the implant (60, 70, 80) can be pushed in a prepared cavity (61) between adjacent vertebrae (17, 18) by being guided via the longitudinal grooves (15) and the longitudinal keys (16, 21, 22), respectively, of the guide instrument (10, 20) cooperating with the corresponding keys (66, 67, 75) and/or grooves (76, 85) of the implant (60, 70, 80).

2. Implantation system according to claim 1, wherein the implant (60, 80) is in the form of a rectangular block or in the form of a cylinder.

3. Implantation system according to claim 1 or 2, wherein the implant (70) is U-shaped, and wherein the grooves (76) and/or the keys (75) are formed on its free U-limbs (71).

4. Implantation system according to one of claims 1 to 3, wherein the guide instrument (10, 20) is designed in form of two guide rails (11, 12), which are arranged parallel to each other and which form one component, or is in form of a cylindrical tube (20).

5. Implantation system according to one of claims 1 to 4, wherein longitudinal keys (16) on the guide instrument (10) are arranged to be exchangeable.

## Revendications

1. Système d'implantation, comprenant un implant (60, 70, 80) pour la fusion vertébrale, qui est pourvu sur son côté extérieur de languettes (66, 67, 75) et/ou de rainures (76, 85), qui s'étendent dans la direction longitudinale de l'implant (60, 70, 80), et comprenant un instrument de guidage (10, 20) oblong, qui comporte des rainures longitudinales (15) ou des languettes longitudinales (16, 21, 22) adaptées aux languettes (66, 67, 75) et/ou aux rainures (76, 85) de l'implant (60, 70, 80), système dans lequel l'implant (60, 70, 80) peut, en étant guidé au moyen de ses languettes (66, 67, 75) et/ou rainures (76, 85) par les rainures longitudinales (15) ou languettes longitudinales (16, 21, 22) associées de l'instrument de guidage (10, 20), être enfilé dans une cavité (61) préparée entre des corps vertébraux (17, 18) voisins.

2. Système d'implantation selon la revendication 1, dans lequel l'implant (60, 80) est réalisé de forme parallélépipédique ou cylindrique.

3. Système d'implantation selon la revendication 1 ou 2, dans lequel l'implant (70) est réalisé en forme de U, et les rainures (76) et/ou les languettes (75) sont formées sur ses branches libres (71) du U.

4. Système d'implantation selon l'une quelconque des revendications 1 à 3, dans lequel l'instrument de guidage (10, 20) est réalisé sous la forme de deux rails de guidage (11, 12) s'étendant parallèlement entre eux et réunis en une pièce, ou sous la forme d'un tube cylindrique (20).

5. Système d'implantation selon l'une quelconque des revendications 1 à 4, dans lequel les languettes longitudinales (16) sont disposées avec possibilité de remplacement sur l'instrument de guidage (10).
